(19) Europäisches Patentamt
European Patent Office
Office européen des brevets

(11) Publication number: **0 307 041 B1**

# (12) EUROPEAN PATENT SPECIFICATION

(45) Date of publication of patent specification: **15.12.93**

(51) Int. Cl.[5]: **C07D 211/46**, C08K 5/34

(21) Application number: **88201890.6**

(22) Date of filing: **05.09.88**

The file contains technical information submitted after the application was filed and not included in this specification

## (54) Sterically hindered amino compounds and processes for their preparation.

(30) Priority: **11.09.87 IT 2189087**

(43) Date of publication of application:
**15.03.89 Bulletin 89/11**

(45) Publication of the grant of the patent:
**15.12.93 Bulletin 93/50**

(84) Designated Contracting States:
**AT BE CH DE ES FR GB GR LI LU NL SE**

(56) References cited:
**DE-A- 2 258 752**
**US-A- 3 640 928**

(73) Proprietor: **ENIRICERCHE S.p.A.**
**Corso Venezia 16**
**I-20121 Milan(IT)**

Proprietor: **ENICHEM SYNTHESIS S.p.A.**
**Via Ruggero Settimo 55**
**I-90139 Palermo(IT)**

(72) Inventor: **Roggero, Arnaldo**
**Via Libertà 72**
**I-20097 San Donato Milanese (MI)(IT)**
Inventor: **Bertolini, Guglielmo**
**Via Parco Vecchio 30/3**
**I-27100 Pavia(IT)**

(74) Representative: **Roggero, Sergio et al**
**Ing. Barzanò & Zanardo Milano S.p.A.**
**Via Borgonuovo 10**
**I-20121 Milano (IT)**

**EP 0 307 041 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to sterically hindered amino compounds, processes for their preparation and their use in the stabilization of organic polymers.

It is well known that organic polymers undergo degradation with time due to exposure to atmospheric agents and in particular to ultraviolet radiation. To counteract this degradation it is usual in the art to introduce into organic polymers small quantities of stabilizer compounds generally consisting of sterically hindered amines, as described in USA patents 3,640,928, 3,840,494 and 4,046,731.

In stabilizing organic compounds with sterically hindered amino compounds there are problems of compatibility between the stabilizer and polymer, and problems due to extractability of the stabilizer from the polymer, this latter phenomenon depending on the molecular weight of the stabilizer used. Certain stabilizer compounds have therefore been proposed having a relatively simple structure but able to convert spontaneously into compounds of complex resinous type in the organic polymer in which they are incorporated, and others of polymer type have been proposed having a similar structure to the polymer to be stabilized, as described for example in European patent applications Pubblication Nos. 162,532 - 218,296 and 248,494.

There is a need for light-stabilizing compounds for polymer materials which besides being thermally stable and compatible with the organic polymer possess at the same time the characteristics of low extractability from the polymer and good mobility within the polymer structure. In this respect, stabilizers possessing such characteristics are particularly suitable for protecting articles of polymer construction having a large surface area, and general polymer-constructed articles requiring a high local stabilizer concentration because of particularly exposed local regions, compared with other less exposed regions.

There is also a need for stabilizing compounds which besides having the aforesaid characteristics can be prepared simply and economically from raw materials which are either commercially available or easily synthesized.

It has now been found that such requirements can be satisfied by the sterically hindered amino compounds of the present invention, which are definable by the general formula (I):

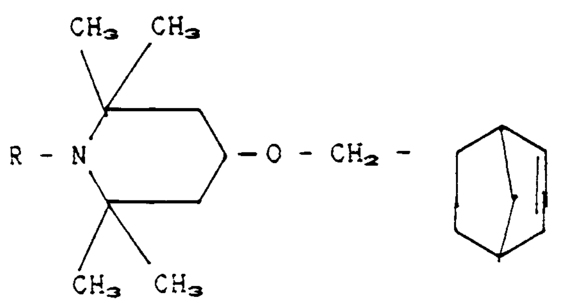

(I)

where R represents a hydrogen atom, a $C_1$-$C_{18}$ alkyl radical or a $C_1$-$C_{18}$ aryl radical, the alkyl radical being linear or branched. In the preferred embodiment, in general formula (I) R represents a hydrogen atom or the methyl or benzyl radical.

Consequently, sterically hindered amino compounds preferred for the purposes of the present invention are:

4-oxymethylene-[2′-(norborn-5′-enyl)]-(2,2,6,6-tetramethylpiperidine);
4-oxymethylene-[2′-(norborn-5′-enyl)]-(2,2,6,6-tetramethyl-N-methyl-piperidine); and
4-oxymethylene-[2′-(norborn-5′-enyl)]-(2,2,6,6-tetramethyl-N-benzyl-piperidine).

The sterically hindered amino compounds (I) can be obtained by the Diels-Adler reaction, by reacting cyclopentadiene (II) with a 4-oxy-allyl-2,2,6,6-tetramethyl-piperidine (III):

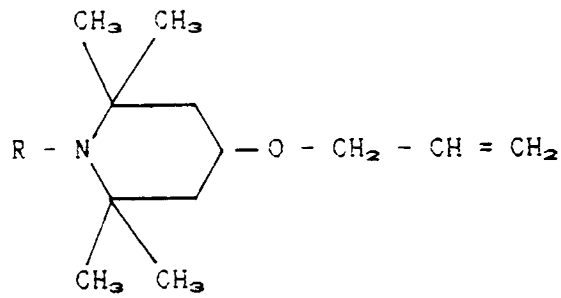

(III)

where R has the aforesaid meaning.

The reaction is conveniently conducted in the liquid phase in an inert organic solvent at a temperature of between 100 °C and 200 °C and preferably of the order of 170 °C at greater than atmospheric pressure, using an excess of compound (II) over its stoichiometric requirement with respect to compound (III), and generally with a ratio of compound (II) to compound (III) of between 1.1:1 and 2.0:1.

Inert organic solvents suitable for this purpose are preferably aliphatic and cycloaliphatic hydrocarbon solvents such as hexane, heptane and cyclohexane.

Under these conditions the time required for the reaction to go to completion is of the order of 1-3 hours.

On termination of the reaction the sterically hindered amino compounds can be recovered from the reaction mixture by normal methods.

In the case of sterically hindered amino compounds (I) in which R is other than hydrogen, the procedure can be carried out by firstly reacting cyclopentadiene with 4-oxy-allyl-2,2,6,6-tetramethyl-piperidine to give 4-oxymethylene-[2′-(norborn-5′-enyl)]-(2,2,6,6-tetramethyl-piperidine) and reacting this latter with an alkyl or alkyl-aryl halide (such as methyl iodide or benzyl chloride) to give the final desired product. This latter reaction is normally conducted in the presence of a hydrohalogen acid acceptor.

The sterically hindered compounds of the present invention are easily mixed and homogenized with organic polymers and have compatibility, mobility and non-extractability especially with regard to styrene polymers and polyolefins.

Thus, according to a further aspect, the present invention relates to compositions stable towards the degradative action of ultraviolet light, which comprise an organic polymer, especially a styrene polymer or a polyolefin, such as polyethylene or polypropylene, and a quantity of sterically hindered amino compound (I) such that the composition contains an active nitrogen quantity of between 0.005% and 0.02% by weight, and preferably of the order of 0.015% by weight.

The term "active nitrogen" signifies the nitrogen contributed by the sterically hindered amino group.

The stabilized polymer compositions of the present invention can be prepared by any known method used for mixing and homogenizing a polymer with a stabilizer.

The experimental examples given hereinafter further illustrate the present invention

EXAMPLE 1

Preparation of 4-oxymethylene-[2′-(norborn-5′-enyl)]-(2,2,6,6-tetramethyl-piperidine):

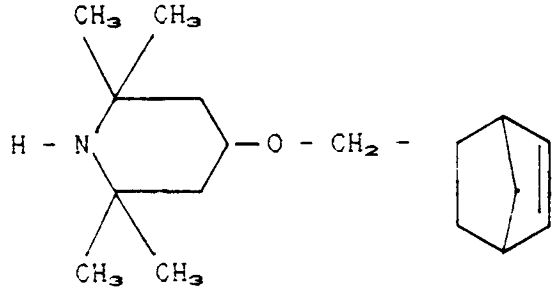

A mixture of 29.0 g (0.15 moles) of 4-oxy-allyl-2,2,6,6-tetramethyl-piperidine and 18,5 ml (0.225 moles) of cyclopentadiene dissolved in 35 ml of anhydrous n-heptane is drawn under vacuum into a 200 ml

autoclave provided with a magnetic stirrer.

The autoclave is immersed in an oil bath heated to 170 ° C. The reaction is conducted under stirring for three hours.

The crude reaction product is subjected to fractional distillation. Cyclopentadiene, n-heptane and dicyclopentadiene are firstly removed under slight vacuum; 4-oxy-allyl-2,2,6,6-tetramethyl-piperidine is then removed in an unaltered state (boiling point 45 ° C/0.5 torr) and finally the compound of the title (boiling point 94 ° C/0.1 torr) is collected in a quantity of 13 g (molar yield 33% with respect to 4-oxy-allyl-2,2,6,6-tetramethyl-piperidine) with a purity of 94% (determined by gas chromatography).

The compound of the title is characterised by mass spectrometry and IR and NMR spectrography. The data deriving from the IR and NMR characterisation are given in Tables I and II respectively.

EXAMPLE 2

Preparation of 4-oxymethylene-[2′-(norborn-5′-enyl)]-(2,2,6,6-tetramethyl-N-methyl-piperidine):

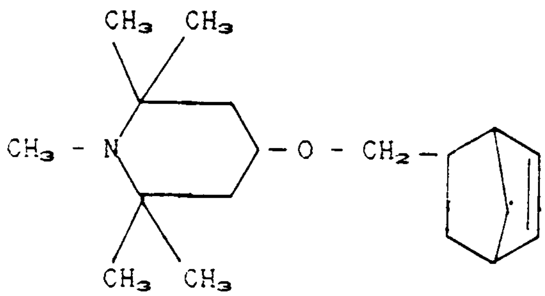

Method A

A mixture of 21.5 g (0.102 moles) of 4-oxy-allyl-2,2,6,6-tetramethyl-N-methyl-piperidine and 12.6 ml (0.153 moles) of cyclopentadiene dissolved in 35 ml of anhydrous n-heptane is drawn under vacuum into a 200 ml autoclave provided with a magnetic stirrer. The autoclave is immersed in an oil bath heated to 170 ° C. The reaction is conducted under stirring for three hours.

The crude reaction product is subjected to fractional distillation. Cyclopentadiene, n-heptane and dicyclopentadiene are firstly removed under slight vacuum; 4-oxy-allyl-2,2,6,6-tetramethyl-piperidine is then removed in an unaltered state (boiling point 88 ° C/0.1 torr) and finally the compound of the title (boiling point 143 ° C/0.1 torr) is collected in a quantity of 9.7 g (molar yield 34% with respect to 4-oxy-allyl-2,2,6,6-tetramethyl-N-methyl-piperidine) with a purity of 95% (determined by gas chromatography).

The compound of the title is characterised by mass spectrometry and IR and NMR spectrography. The data deriving from the IR and NMR characterisation are given in Tables I and II respectively.

Method B

10 g (0.038 moles) of 4-oxymethylene-[2′-(norborn-5′-enyl)]-(2,2,6,6-tetramethyl-piperidine) (prepared as described in Example 1) dissolved in 100 ml of methanol are fed into a 250 ml flask provided with a mechanical stirrer and reflux condenser.

37.5 g (0.264 moles) of methyl iodide and 11.5 g (0.287 moles) of sodium hydroxide are added. The reaction is conducted under reflux conditions for 8 hours and at the end of this time gas chromatography analysis shows that the reaction is complete.

The crude reaction product is treated with water and extracted with ethyl ether. The solvent and volatile substances are removed from the organic phase by evaporation under vacuum to recover 9.1 g (yield 86.4%) of the compound of the title, with characteristics entirely similar to those of the compound prepared by method (A).

EXAMPLE 3

Preparation of 4-oxymethylene-[2′-(norborn-5′-enyl)]-(2,2,6,6-tetramethyl-N-benzyl-piperidine):

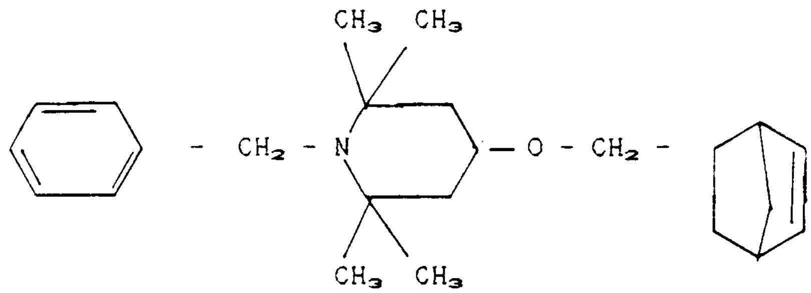

Method A

A mixture of 23 g (0.080 moles) of 4-oxy-allyl-2,2,6,6- tetramethyl-N-benzyl-piperidine and 9,9 ml (0.12 moles) of cyclopentadiene dissolved in 35 ml of anhydrous n-heptane is drawn under vacuum into a 200 ml autoclave provided with a magnetic stirrer. The autoclave is immersed in an oil bath heated to 170 ° C.

The reaction is conducted under stirring for three hours.

The crude reaction product is subjected to fractional distillation. After removing the solvent and unreacted compounds, the compound of the title is recovered as distillation residue in the form of a viscous oil with a molar yield of 37% with respect to 4-oxy-allyl-2,2,6,6-tetramethyl-N-benzyl-piperidine with a purity of 97.6% (determined by gas chromatography).

The compound of the title is characterised by mass spectrometry and IR and NMR spectrography. The data deriving from the IR and NMR characterisation are given in Tables I and II respectively.

Method B

5.0 g (0.019 moles) of 4-oxymethylene-[2′-(norborn-5′-enyl)]-(2,2,6,6-tetramethyl-piperidine) (prepared as described in Example 1) and 6.5 g (0.051 moles) of benzyl chloride are fed into a flask provided with a mechanical stirrer and reflux condenser. The reaction is conducted for 5 hours at 150 ° C and at the end of this time gas chromatography analysis shows that the reaction is complete.

The crude reaction product is treated in the same manner as in method (A) to recover 6.04 g (yield 90%) of the compound of the title, with characteristics entirely similar to those of the compound prepared by method (A).

TABLE I

IR absorption characteristics

| Compound Ex. No. | ν N-H | ν =C-H | ν C=C | ν C=C | δ symm $CH_3$ | $CH_3$ r | β =C-H | ν C-O | γ =C-H |
|---|---|---|---|---|---|---|---|---|---|
| (1) | 3135 | 3130 | 1643 | - | 1374 | 1274 | - | 1094 | 718 |
| | | 3055 | | | 1363 | 1190 | | | |
| (2) | - | 3130 | - | - | 1372 | 1252 | - | 1094 | 717 |
| | | 3050 | | | 1358 | 1180 | | | |
| (3) | - | 3195 | - | 1602 | 1381 | 1258 | 1171 | 1096 | 740 |
| | | 3100 | | 1584 | 1368 | 1180 | 1025 | | 719 |
| | | 3084 | | 1493 | | | | | 696 |
| | | 3060 | | | | | | | |
| | | 3025 | | | | | | | |

TABLE II

Chemical shifts (ppm by TMS)

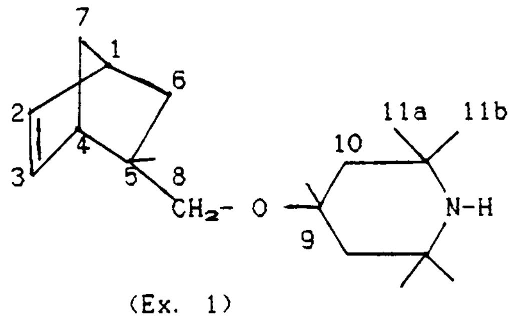

(Ex. 1)

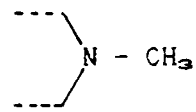

(Ex. 2)

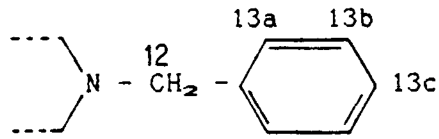

(Ex. 3)

| Compound<br>Ex. No. | 1 | 2 | 3 | 4 | 5 | 6 | 7 | 8 | 9 | 10 | 11a | 11b | 12 | 13a | 13b | 13c |
|---|---|---|---|---|---|---|---|---|---|---|---|---|---|---|---|---|
| (1) | 2,8 | 5,96 | 6,15 | 2,93 | 2,32 | 1,86 | 1,3 | 3,17 | 3,6 | 1,8 | 1,1 | 1,14 | - | - | - | - |
| (2) | 2,76 | 5,95 | 6,12 | 2,9 | 2,35 | 1,85 | 1,3 | 3,1 | 3,45 | 1,83 | 1,0 | 1,15 | - | - | - | - |
| (3) | 2,8 | 5,96 | 6,15 | 2,93 | 2,35 | 1,88 | 1,3 | 3,19 | 3,6 | 1,8 | 1,1 | 1,14 | 3,8 | 7,42 | 7,25 | 7,1 |

The compounds prepared in Examples 1, 2 and 3 are added to the commercial polyethylene RIBLENE® A42CL of Messrs. ENICHEM in a quantity such as to provide an active N content of 0.015% by weight in the polymer.

Homogenization is obtained by two extrusion passes through an MFI apparatus.

The compositions are pressed to form films of 110μ thickness.

The films thus obtained are subjected to an accelerated ageing test in UV CON with irradiation cycles of 8 hours at 60 °C with a fluorescent lamp (UV radiation between 280 and 350 nm) and 4 hours of condensation in the dark at 40 °C.

The degradation is evaluated on the basis of the increase in the carbonyl index, which is measured by IR spectroscopy. The results of the test are given in the graph of the Figure, in which the horizontal axis represents the residence time in UV CON in hours and the vertical axis represents the carbonyl index.

In this graph:

◊—◊ is the curve for polyethylene film as such;

O—O is the curve for polyethylene film containing the compound of Example 2;

□—□ is the curve for polyethylene film containing the compound of Example 3; and

Δ—Δ is the curve for polyethylene film containing the compound of Example 1.

## Claims

**Claims for the following Contracting States : AT, BE, CH, DE, FR, GB, GR, LI, LU, NL, SE**

1. Sterically hindered amino compounds definable by the general formula (I):

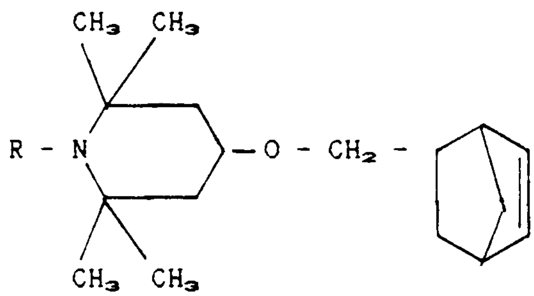

(I)

where R represents a hydrogen atom, a $C_1$-$C_{18}$ alkyl radical or a $C_1$-$C_{18}$ aryl radical, the alkyl radical being linear or branched.

2. Compounds as claimed in claim 1, characterised in that in formula (I) R represents a hydrogen atom, the methyl radical or the benzyl radical.

3. A process for preparing the sterically hindered amino compounds claimed in claim 1, characterised by reacting cyclopentadiene (II) with a 4-oxy-allyl-2,2,6,6-tetramethyl-piperidine (III):

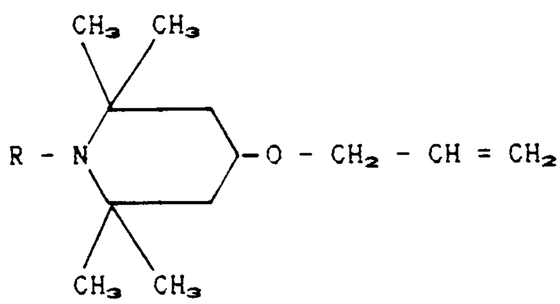

(III)

where R has the meaning given in claim 1;
and operating in the liquid phase in an inert organic solvent at a temperature of between 100 °C and 200 °C at greater than atmospheric pressure, using an excess of compound (II) over its stoichiometric requirement with respect to compound (III).

4. A process as claimed in claim 3, characterised in that the inert organic solvent is chosen from aliphatic and cycloaliphatic hydrocarbon solvents.

5. A process as claimed in claim 3, characterised by operating at a temperature of the order of 170 °C.

6. A process as claimed in claim 3, characterised by operating with a molar ratio of compound (II) to compound (III) of between 1.1:1 and 2.0:1.

7. Stabilized polymer compositions characterised by comprising an organic polymer and a stabilizing quantity of a sterically hindered amino compound claimed in claim 1 or 2.

8. Stabilized polymer compounds as claimed in claim 7, characterised in that the organic polymer is chosen from polyolefins and styrene polymers.

**Claims for the following Contracting State : ES**

**1.** A process for preparing sterically hindered amino compounds definable by the general formula (I):

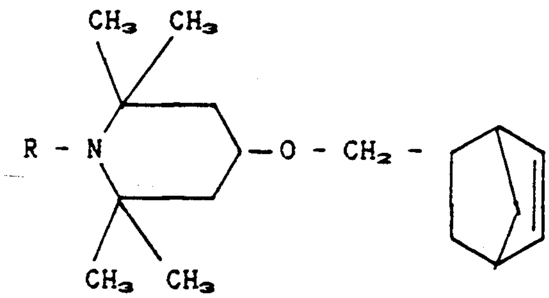

(I)

where R represents a hydrogen atom, a $C_1$-$C_{18}$ alkyl radical or a $C_1$-$C_{18}$ aryl radical, the alkyl radical being linear or branched, characterised by reacting cyclopentadiene (II) with a 4-oxy-allyl-2,2,6,6-tetramethylpiperidine (III):

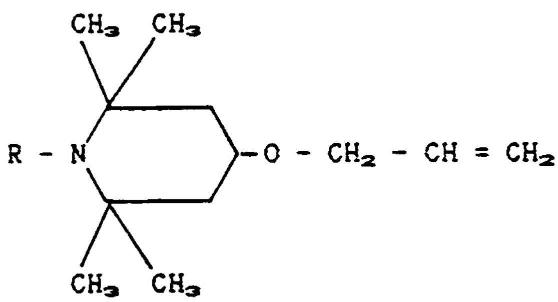

(III)

where R have the above indicated meaning;
and operating in the liquid phase in an inert organic solvent at a temperature of between 100 °C and 200 °C at greater than atmospheric pressure, using an excess of compound (II) over its stoichiometric requirement with respect to compound (III).

**2.** A process as claimed in claim 1, characterised in that in formula (I), R represents a hydrogen atom, the methyl radical or the benzyl radical.

**3.** A process as claimed in claim 1, characterised in that the inert organic solvent is chosen from aliphatic and cycloaliphatic hydrocarbon solvents.

**4.** A process as claimed in claim 1, characterised by operating at a temperature of the order of 170 °C.

**5.** A process as claimed in claim 1, characterised by operating with a molar ratio of compound (II) to compound (III) of between 1.1:1 and 2.0:1.

**6.** A process for preparing stabilized polymer compositions characterised by the step of combining together an organic polymer and a stabilizing quantity of a sterically hindered amino compound as claimed in claim 1 or 2.

**7.** A process as claimed in claim 6, characterised in that the organic polymer is chosen from polyolefins and styrene polymers.

**Patentansprüche**
**Patentansprüche für folgende Vertragsstaaten : AT, BE, CH, DE, FR, GB, GR, LI, LU, NL, SE**

1. Sterisch gehinderte Aminoverbindungen, definierbar durch die allgemeine Formel (I):

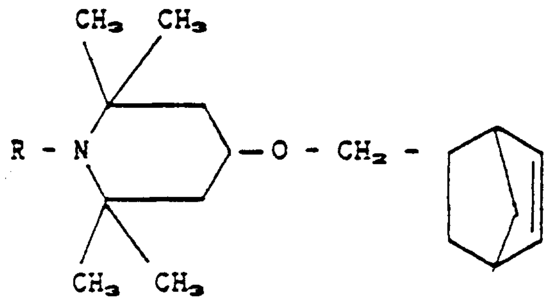

(I),

worin R ein Wasserstoffatom, einen $C_1$-$C_{18}$Alkylrest oder einen $C_1$-$C_{18}$Arylrest darstellt, wobei der Alkylrest linear oder verzweigt ist.

2. Verbindung nach Anspruch 1, dadurch gekennzeichnet, daß in der Formel (I) R ein Wasserstoffatom, den Methylrest oder den Benzylrest darstellt.

3. Verfahren zur Herstellung der sterisch gehinderten Aminoverbindungen nach Anspruch 1, gekennzeichnet durch Umsetzen von Cyclopentadien (II) mit einem 4-Oxy-allyl-2,2,6,6-tetramethylpiperidin (III):

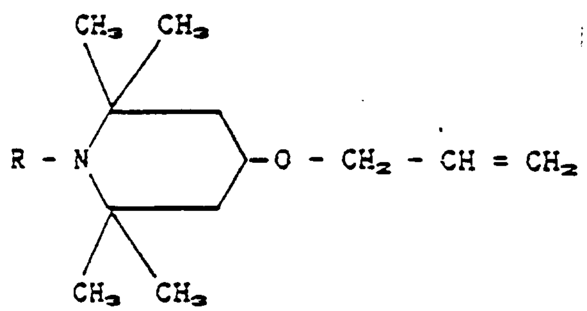

(III),

worin R die in Anspruch 1 angeführte Bedeutung besitzt;
und durch Arbeiten in der flüssigen Phase in einem inerten organischen Lösungsmittel bei einer Temperatur von 100 °C bis 200 °C über Atmosphärendruck, wobei eine gegenüber dem stöchiometrischen Erfordernis im Hinblick auf die Verbindung (III) überschüssige Menge an Verbindung (II) angewandt wird.

4. Verfahren nach Anspruch 3, dadurch gekennzeichnet, daß das inerte organische Lösungsmittel unter aliphatischen und cycloaliphatischen Kohlenwasserstofflösungsmitteln ausgewählt ist.

5. Verfahren nach Anspruch 3, dadurch gekennzeichnet, daß bei einer Temperatur in der Größenordnung von 170 °C gearbeitet wird.

6. Verfahren nach Anspruch 3, dadurch gekennzeichnet, daß mit einem Molverhältnis von Verbindung (II) zu Verbindung (III) von 1,1:1 bis 2,0:1 gearbeitet wird.

7. Stabilisierte Polymerzusammensetzungen,dadurch gekennzeichnet, daß sie ein organisches Polymer und eine stabilisierende Menge einer sterisch gehinderten Aminoverbindung nach Anspruch 1 oder 2 enthalten.

8. Stabilisierte Polymerverbindungen nach Anspruch 7, dadurch gekennzeichnet, daß das organische Polymer unter Polyolefinen und Styrolpolymeren ausgewählt ist.

**Patentansprüche für folgenden Vertragsstaat : ES**

1. Verfahren zur Herstellung sterisch gehinderter Aminoverbindungen, welche durch die allgemeine Formel (I):

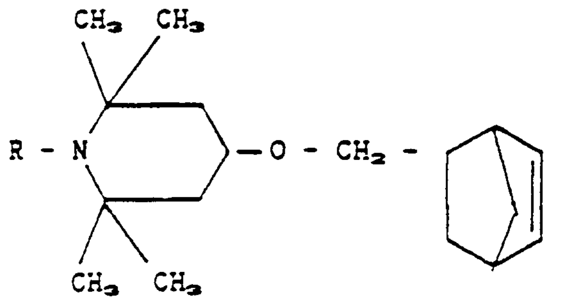

(I)

definierbar sind,worin R ein Wasserstoffatom,einen $C_1$-$C_{18}$Alkylrest oder einen $C_1$-$C_{18}$Arylrest darstellt, wobei der Alkylrest linear oder verzweigt ist, gekennzeichnet durch Umsetzen von Cyclopentadien (II) mit einem 4-Oxy-allyl-2,2,6,6-tetramethylpiperidin (III):

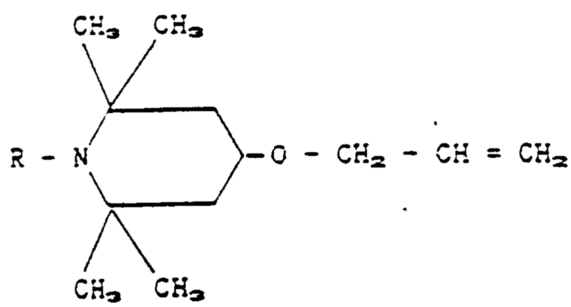

(III),

worin R die vorstehend angeführte Bedeutung besitzt;
und durch Arbeiten in der flüssigen Phase in einem inerten organischen Lösungsmittel bei einer Temperatur von 100 °C bis 200 °C über Atmosphärendruck, wobei eine gegenüber dem stöchiometrischen Erfordernis im Hinblick auf die Verbindung (III) überschüssige Menge an Verbindung (II) angewandt wird.

2. Verfahren nach Anspruch 1, dadurch gekennzeichnet, daß in der Formel (I) R ein Wasserstoffatom, den Methylrest oder den Benzylrest darstellt.

3. Verfahren nach Anspruch 1, dadurch gekennzeichnet, daß das inerte organische Lösungsmittel unter aliphatischen und cycloaliphatischen Kohlenwasserstofflösungsmitteln ausgewählt ist.

4. Verfahren nach Anspruch 1, dadurch gekennzeichnet, daß bei einer Temperatur in der Größenordnung von 170 °C gearbeitet wird.

5. Verfahren nach Anspruch 1, dadurch gekennzeichnet, daß mit einem Molverhältnis von Verbindung (II) zu Verbindung (III) von 1,1:1 bis 2,0:1 gearbeitet wird.

6. Verfahren zur Herstellung stabilisierter Polymerzusammensetzungen, gekennzeichnet durch den Schritt des Vereinigens eines organischen Polymers und einer stabilisierenden Menge einer sterisch gehinderten Aminoverbindung nach Anspruch 1 oder 2.

7. Verfahren nach Anspruch 6, dadurch gekennzeichnet, daß das organische Polymer unter Polyolefinen und Styrolpolymeren ausgewählt ist.

**Revendications**

**Revendications pour les Etats contractants suivants : AT, BE, CH, DE, FR, GB, GR, LI, LU, NL, SE**

**1.** Composés aminés stériquement encombrés, définissables par la formule générale (I) :

(I) 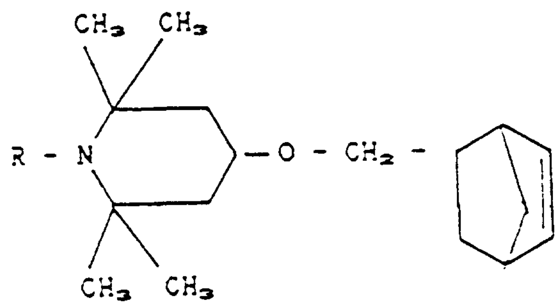

dans laquelle R représente un atome d'hydrogène, un groupe alkyle en $C_1$-$C_{18}$ ou un groupe aryle en $C_1$-$C_{18}$, le groupe alkyle étant linéaire ou ramifié.

**2.** Composés conformes à la revendication 1, caractérisés en ce que, dans la formule (I), R représente un atome d'hydrogène, un groupe méthyle ou un groupe benzyle.

**3.** Procédé de préparation des composés aminés stériquement encombrés conformes à la revendication 1, caractérisé en ce qu'on fait réagir du cyclopentadiène (II) avec une 4-allyloxy-2,2,6,6-tétraméthylpipéridine de formule (III) :

(III) 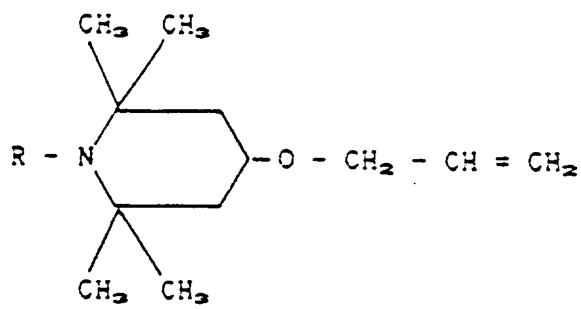

dans laquelle R a la signification indiquée dans la revendication 1,
en opérant en phase liquide, dans un solvant organique inerte, à une température située entre 100 °C et 200 °C et sous une pression supérieure à la pression atmosphérique, et en utilisant plus de composé (II) que ce qu'exige la stoechiométrie, par rapport au composé (III).

**4.** Procédé conforme à la revendication 3, caractérisé en ce que le solvant organique inerte est choisi parmi les solvants hydrocarbonés aliphatiques ou cycloaliphatiques.

**5.** Procédé conforme à la revendication 3, caractérisé en ce que l'on opère à une température de l'ordre de 170 °C.

**6.** Procédé conforme à la revendication 3, caractérisé en ce que l'on opère avec un rapport molaire du composé (II) au composé (III) situé entre 1,1/1 et 2,0/1.

**7.** Compositions de polymère stabilisé, caractérisées en ce qu'elles contiennent un polymère organique et une quantité stabilisatrice d'un composé aminé stériquement encombré conforme à la revendication 1 ou 2.

**8.** Compositions de polymère stabilisé conformes à la revendication 7, caractérisées en ce que le polymère organique est choisi parmi les polyoléfines et les polymères du styrène.

**Revendications pour l'Etat contractant suivant : ES**

1. Procédé de préparation de composés aminés stériquement encombrés, définissables par la formule générale (I) :

(I)

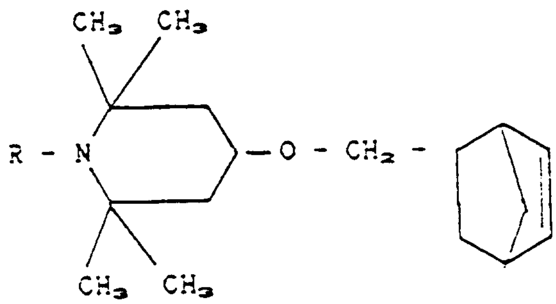

dans laquelle R représente un atome d'hydrogène, un groupe alkyle en $C_1$-$C_{18}$ ou un groupe aryle en $C_1$-$C_{18}$, le groupe alkyle étant linéaire ou ramifié,
caractérisé en ce qu'on fait réagir du cyclopentadiène (II) avec une 4-allyloxy-2,2,6,6-tétraméthylpipéridine de formule (III) :

(III)

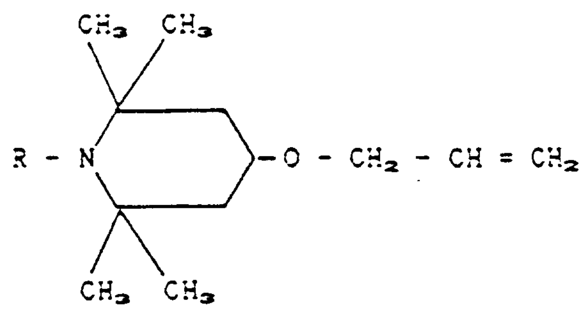

dans laquelle R a la signification indiquée ci-dessus,
en opérant en phase liquide, dans un solvant organique inerte, à une température située entre 100 °C et 200 °C et sous une pression supérieure à la pression atmosphérique, et en utilisant plus de composé (II) que ce qu'exige la stoechiométrie, par rapport au composé (III).

2. Procédé conforme à la revendication 1, caractérisé en ce que, dans la formule (I), R représente un atome d'hydrogène, un groupe méthyle ou un groupe benzyle.

3. Procédé conforme à la revendication 1, caractérisé en ce que le solvant organique inerte est choisi parmi les solvants hydrocarbonés aliphatiques ou cycloaliphatiques.

4. Procédé conforme à la revendication 1, caractérisé en ce que l'on opère à une température de l'ordre de 170 °C.

5. Procédé conforme à la revendication 1, caractérisé en ce que l'on opère avec un rapport molaire du composé (II) au composé (III) situé entre 1,1/1 et 2,0/1.

6. Procédé de préparation de compositions de polymère stabilisé, caractérisé par l'étape consistant à mélanger un polymère organique et une quantité stabilisatrice d'un composé aminé stériquement encombré préparé conformément à la revendication 1 ou 2.

7. Procédé conforme à la revendication 6, caractérisé en ce que le polymère organique est choisi parmi les polyoléfines et les polymères du styrène.

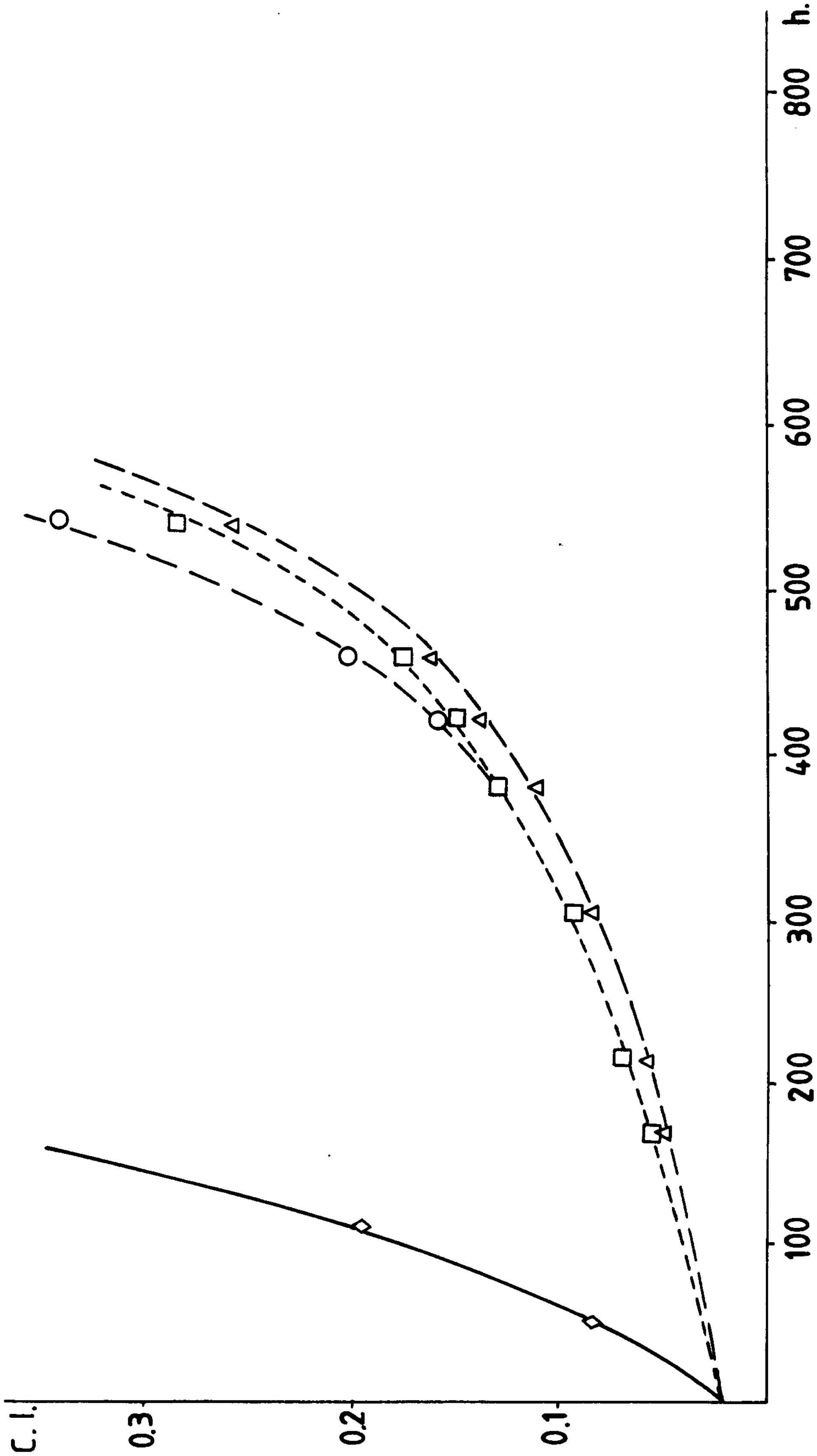
C. I.
0.3
0.2
0.1
100
200
300
400
500
600
700
800
h.